# EUROPEAN PATENT SPECIFICATION

(11) **EP 2 685 902 B1**
(45) Date of publication and mention of the grant of the patent: **17.12.2014**
(21) Application number: 12717855.6
(22) Date of filing: 15.03.2012
(51) Int. Cl.: A61B 8/08, A61B 5/00

(54) **ULTRASOUND AMBULATORY DEVICE FOR THE DIAGNOSIS OF GASTRO-ESOPHAGEAL REFLUX DISEASE**
AMBULANTES ULTRASCHALLGERÄT ZUR DIAGNOSE VON GASTROÖSOPHAGEALEM REFLUX
DISPOSITIF AMBULATOIRE À ULTRASONS POUR DIAGNOSTIQUER UN REFLUX GASTRO-OESPOPHAGIEN PATHOLOGIQUE

(30) Priority: 15.03.2011 IT BO20110123
(43) Date of publication of application: 22.01.2014
(73) Proprietor: Medica S.p.A., 41036 Medolla (IT)
(72) Inventor: EMILIANI, Sergio, 41036 Medolla (IT); BAGNOLI, Davide, 41036 Medolla (IT); BALDI, Fabio, 41036 Medolla (IT); MOLINAZZI, Nicola, 41036 Medolla (IT)
(74) Representative: Boggio, Luigi
(86) International application number: PCT/IB2012/051257
(87) International publication number: WO 2012/123918

(56) References cited:
- US-A1- 2005 124 888
- US-A1- 2005 182 342
- HIRSCH W ET AL: "Color doppler in the diagnosis of the gastroesophageal reflux in children: comparison with pH measurements and B-mode ultrasound", PEDIATRIC RADIOLOGY, SPRINGER VERLAG, DE, vol. 26, no. 3, 1 January 1996 (1996-01-01), pages 232-235, XP009149904, ISSN: 0301-0449
- HYE SUK JANG ET AL: "Correlation of color Doppler sonographic findings with pH measurements in gastroesophageal reflux in children", JOURNAL OF CLINICAL ULTRASOUND, vol. 29, no. 4, 1 May 2001 (2001-05-01), pages 212-217, XP055001936, ISSN: 0091-2751, DOI: 10.1002/jcu.1022
- S. J. WESTRA ET AL: "Ultrasound diagnosis of gastroesophageal reflux and hiatal hernia in infants and young children", Journal of clinical ultrasound, vol. 18, No. 6, 1 July 1990 (1990-07-01), pages 477-485, XP055002041, Retrieved from the Internet: URL:http://onlinelibrary.wiley.com/store/1 0.1002/jcu.1870180605/asset/1870180605_ftp .pdf?v=1&t=gprxyt5o&s=0a24958ce016fd139946 41403f50d0ceaa35209e [retrieved on 2011-07-06]

## Description

### TECHNICAL FIELD

The present invention concerns an ambulatory device for the diagnosis of gastro-esophageal reflux disease.

### BACKGROUND ART

In the industrialised countries disorders caused by gastro-esophageal reflux disease, often abbreviated to GERD, are increasingly widespread. Gastro-esophageal reflux is a pathology caused by the reflux of acid material from the stomach towards the esophagus via a valve, the lower esophageal sphincter, which in normal conditions should allow the passage of food and liquids only from the esophagus towards the stomach. Gastro-esophageal reflux is currently increasingly widespread both in the adult population and among infants and children and, if not diagnosed in time and appropriately treated, can degenerate into more serious pathologies.

The most widely used technique for the diagnosis of gastro-esophageal reflux disease is the ambulatory 24-hour gastro-esophageal pH test. A device is connected to the patient comprising a recorder and a single or dual-channel nasogastric probe which is positioned in the distal tract of the esophagus and if necessary (in the case of a dual-channel probe) also in the stomach. With this type of technique, it is possible to identify and record the pH of the esophagus (and of the stomach) of a patient over a period of twenty-four hours, in order to detect drops in the pH below a predetermined threshold value (usually pH 4) which identify a reflux of acid material from the stomach towards the esophagus, while the patent carries out his/her normal activities. The device is connected by specialist personnel to the patient who can lead a normal life during the 24-hour period of the examination. At the end of the twenty-four hour period, the probe is removed and the doctor can transfer the values recorded during the twenty-four hour period from the portable recording unit to a control unit, typically a computer, where dedicated software analyses them and produces results useful for diagnosis and treatment.

This ambulatory technique is clearly distinct from other methods in which the patient is connected for the entire duration of the examination to a diagnostic device in an outpatient surgery or in a hospital. These examinations rarely last longer than sixty minutes. In the latter case the expression "stationary" technique is also used.

The ambulatory pH test allows correct diagnosis of gastro-esophageal reflux disease since, during the 24 hour period, a certain number of gastro-esophageal reflux episodes always occur.

Furthermore over the years it has become evident that the reflux episodes are concentrated in certain moments of the day and are greatly influenced by the condition of the patient. It therefore appears clear that the stationary technique is not very effective, since a hospital test lasting sixty minutes may not identify any gastro-esophageal reflux and therefore may not lead to a correct diagnosis of the disease.

The drawback of the ambulatory pH test, however, is that it is a very invasive and uncomfortable method for the patient, in particular for children and infants.

In literature, various non-invasive diagnosis systems for esophageal pathologies are known.

In particular, the patent application US-A1-2005124888 describes a method and equipment for monitoring the esophageal motility of a patient by means of ultrasound images. The equipment described in US-A1-2005124888 is suitable for recording ultrasound images that occupy a large amount of memory and it is therefore not able to perform 24-hour recordings. The equipment described can therefore not be advantageously applied to the diagnosis of gastro-esophageal reflux disease. Furthermore, the monitoring method is not able to automatically recognise the episodes of gastro-esophageal reflux.

The article by Dahnoun N. et al, Medical and Biological Engineering and Computing, Springer, Heildelberg , De LNKD-DOI entitled "Portable Directional Ultrasonic Doppler Blood Velocimeter for Ambulatory Use" (10.1007/BF02441971, vol. 28, no. 5 1 September 1990, pages 471-482) relates to a portable system dedicated to study of the blood vessels. The control unit of the portable system described in the above-mentioned article is aimed at calculating the peak frequency and consequently the speed and direction of the blood flow at a certain moment. In the portable system described in the above-mentioned article, the detection is discrete and, in particular, the portable system is activated at time intervals of approximately two minutes. The portable system described cannot therefore be applied to the diagnosis of gastro-esophageal reflux disease.

The article by Hirsh W. et al, PEDIATRIC RADIOLOGY, SPRINGER VERLAG, DE, 1 January 1996: "Color Doppler in the diagnosis of the gastroesophageal reflux in children: comparison with pH measurements and B-Mode Ultrasound*"* suggests that it is possible to identify gastro-esophageal reflux in children by means of Eco Color Doppler. However, the above-mentioned article does not provide any indication of how the technique of Eco Color Doppler can be successfully applied to measurement over a prolonged period of time.

US2005/182342 describes a diagnostic instrument for prolonged recordings of different signals inside the esophagus, including a doppler signal, which has the purpose of studying different esophageal pathologies including gastro-esophageal reflux. Said device, which forms the closest prior art for the invention, however, does not solve the problem of invasiveness, as it too has to be positioned inside the patient, and does not provide any idea of how the refluxes recorded by the device can then be identified automatically by the device itself. The aim of the present invention is therefore to provide a portable device for the diagnosis of gastro-esophageal reflux disease, which overcomes the drawbacks of the state of the art and which is not invasive and uncomfortable for the patient in order to allow also paediatric use and the performance of prolonged tests with automatic identification of refluxes.

### DISCLOSURE OF INVENTION

According to the present invention, a portable device is provided for the diagnosis of gastro-esophageal reflux disease according to the attached claims.

### BRIEF DESCRIPTION OF THE DRAWINGS

The present invention will now be described with reference to the accompanying drawings which illustrate a non-limiting embodiment example in which:
- figure 1 illustrates a preferred embodiment of the portable device produced in accordance with the present invention;
- figure 2 is a schematic view of application of the portable device of figure 1 on a patient;
- figures 3a to 3c illustrate alternative embodiments of a detail of figure 1.

### BEST MODE FOR CARRYING OUT THE INVENTION

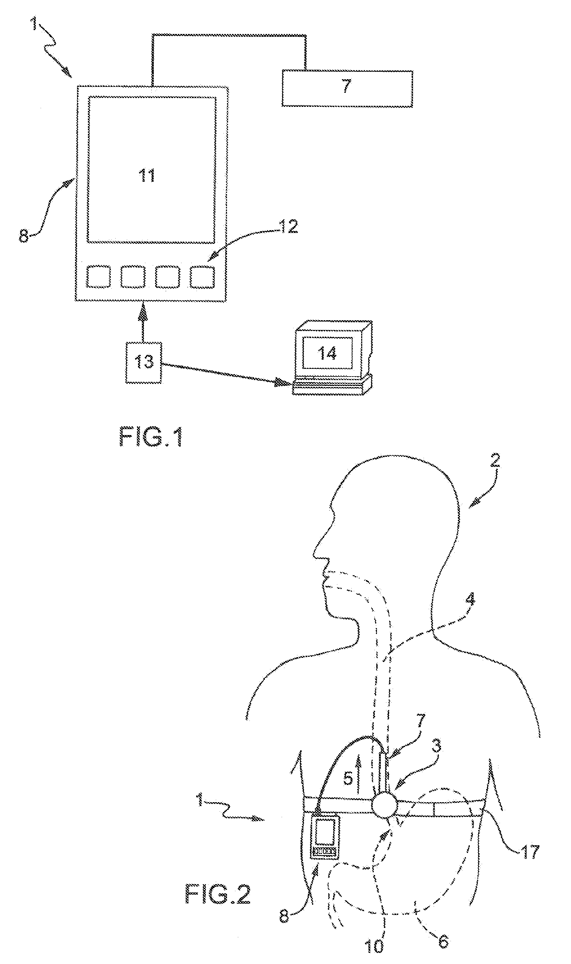

In figure 1, number 1 indicates as a whole an ambulatory device which can be worn by a patient 2 who has to undergo examinations for the diagnosis of gastro-esophageal reflux disease, often abbreviated to GERD.

The portable device 1 for the diagnosis of gastro-esophageal reflux disease is suitable for monitoring the flow of material inside a terminal portion 3 of the esophagus 4 of the patient 2; in particular, the portable device 1 is suitable for recognising the flow of gastric material that moves in the direction indicated by the arrow 5.

The arrow 5 indicates the pathological direction of the flow of gastric material due to reflux from the stomach 6 towards the esophagus 4 of the patient 2; this direction is opposite to the physiological direction of the flow of alimentary and liquid material from the esophagus 4 to the stomach 6.

The portable device 1 for diagnosis comprises a monitoring unit 7 and a recording unit 8.

The recording unit 8 acts as an interface with an operator and/or with the patient 2 who undergoes the examination, it is aimed at storing the data coming from the monitoring unit 7 and at supplying the monitoring unit 7 itself.

According to a preferred variation, the monitoring unit 7 comprises a number of ultrasound probes 9. In particular in the embodiment illustrated in figure 1, one single eco doppler probe 9 is positioned in contact with the thorax of the patient 2 at the level of the lower esophageal sphincter 10 (commonly known as LES), i.e. at the level of the valve located between the terminal portion 3 of the esophagus 4 and the stomach 6 and which opposes the passage of gastric material from the stomach 6 to the esophagus 4.

In healthy patients, the lower esophageal sphincter 10 opens only to allow passage of the bolus from the esophagus 4 towards the inside of the stomach 6.

In patients suffering from gastro-esophageal reflux, functioning of the lower esophageal sphincter 10 is compromised, allowing the gastric material to flow also in the opposite direction, i.e. from the stomach 6 to the esophagus 4.

The recording unit 8 is connected to the monitoring unit 7 and has relatively limited dimensions and a form that allows it to be fixed to the thorax of the patient 2 and be easily carried by the patient 2 for the entire duration of the diagnostic examination of no less than twenty-four hours.

The recording unit 8 comprises a touch-screen display 11 via which, in a preliminary setting and preparation phase of the diagnostic examination, the doctor can enter the data of the patient 2 undergoing the examination and can set the examination parameters, for example the duration and frequency for sampling the data of the patient 2.

The recording unit 8 is furthermore provided with an external user interface keypad 12, via which the patient 2 can indicate the occurrence of particular events in the course of the twenty-four hour period. For example, the patient 2 could indicate via the external keypad the beginning and end of the time intervals dedicated to the intake of food or drink. The changes in position of the patient 2 are recorded automatically by an internal position sensor (not illustrated).

The recording unit 8 furthermore comprises a memory unit 13 positioned inside the recording unit 8 in which the data collected are stored and saved.

The portable device 1 for diagnosis furthermore comprises a data processing unit 14. According to a first variation, the data processing unit 14 is physically separate from the recording unit 8. The memory unit 13 is removable so that, at the end of the twenty-four hour period of the examination, the doctor can remove it from the recording unit 8 and insert it in the data processing unit 14, usually a computer. The data processing unit 14 is equipped with specific data management and processing software which extracts, analyses and processes the data which are collected over the twenty-four hour period in the memory unit 13.

According to a second variation, the data processing unit 14 is integrated in the recording unit 8 and is equipped with specific data management and processing software which extracts, analyses and processes the data which are collected over the twenty-four hour period in the memory unit 13.

In other words, the information stored in the memory unit 13 is analysed to diagnose the gastro-esophageal reflux disease and to evaluate the conditions of the patient 2.

The data processing unit 14 is furthermore aimed at correlating the episodes of gastro-esophageal reflux with the declared activities performed by the patient 2 (intake of food, rest, etc.) and with the symptoms declared by the patient during the twenty-four hour period via the external keypad 12.

Furthermore, the recording unit 8 also comprises a battery pack, able to store and deliver the energy necessary for operation of the monitoring unit 7.

Lastly, the recording unit 8 houses a processor aimed at collecting and storing the data acquired by the monitoring unit 3 and at subsequently transmitting it to the memory unit 13 that stores it.

The ultrasound probe 9 comprises a transducer 15 (i.e. a piezo-element), which has a resonance frequency varying from 1MHz to 10MHz according to the anatomy of the patient 2.

According to a first variation, the ultrasound probe 9 comprises one single transducer 15 (pulsed doppler - PW). According to a second variation, the ultrasound probe 9 comprises a dual transducer 15, or a receiver transducer and a transmitter transducer (continuous doppler - CW).

The dual transducer 15 differs from the single transducer 15 due to the fact that it collects data without distinguishing the depth from which the data come and that it has no limits in measurement of the speed.

The single transducer 15 allows localisation at a predetermined depth of the body of the patient 2 and therefore measurement of the speed within a selected region of the body of the patient 2. Use of the single transducer 15 allows, after a preliminary phase of calibration and setting, measurement of the flow exactly inside the esophagus 4, simplifying the filterings performed by the analysis algorithm to eliminate background noise (as described in further detail below). The depth of the measurement is adapted according to the anatomy of the patient 2.

The ultrasound probe 9 must be applied in direct contact with the body of the patient 2. The point of application must be chosen in order to minimise the acoustic impedance between the transducer 15 and the esophagus 4 of the patient 2. Via a number of experimental tests conducted on different patients 2, it has been established that the optimal position is immediately below the xiphoid process (i.e. immediately below the terminal protuberance constituting the lower part of the sternum).

To calculate the doppler frequency it is of fundamental importance to determine the value of the Doppler angle θ, or angle of attack θ, which represents the angle formed between the forward direction of the gastro-esophageal reflux and the direction of propagation of the ultrasound wave. It was verified that with an angle of attack θ ranging from 30° to 60° it is possible to obtain a good quality signal.

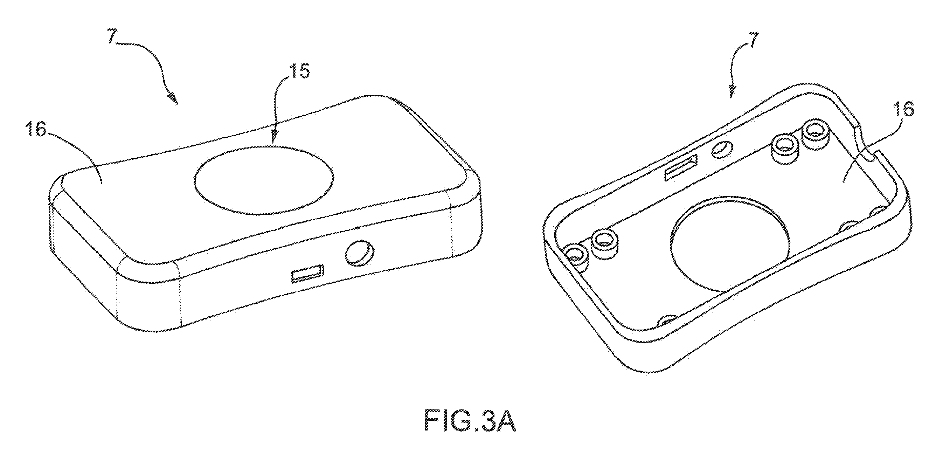

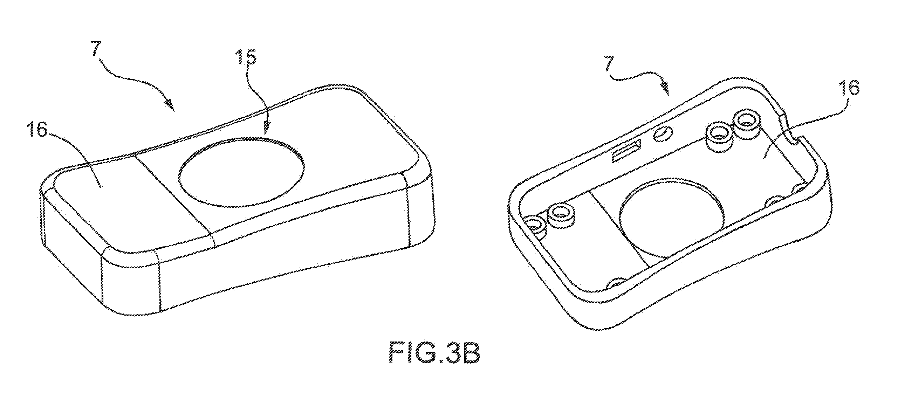

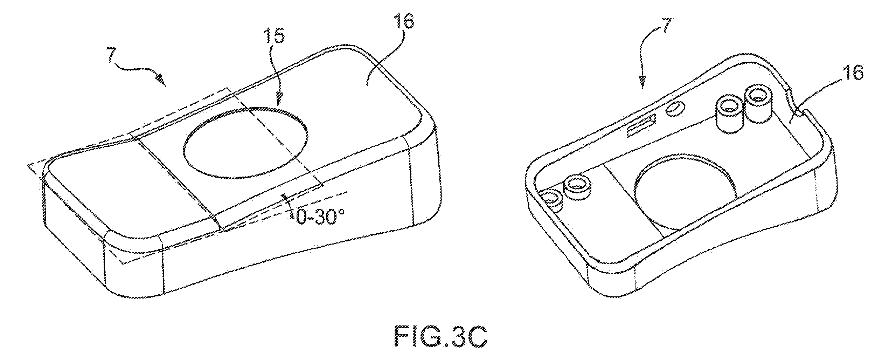

The monitoring unit 7 comprises an outer shell 16 made of plastic. On the shell 16 a seat is obtained to house the transducer 15 in order to maintain the angle of attack θ within the above-mentioned range of optimal values. In view of the considerable anatomical differences there may be between the various patients 2, different types of shells 16 can be used, some of which are illustrated by way of example in figure 3. The difference between the variations illustrated in figure 3 lies in the angle formed between the plane on which the transducer 15 lies and the front plane of the patient 2. The values of this angle are variable and preferably between 0° and 30°.

According to a preferred variation, the outer shell 16 comprises an upper appendix (not illustrated) which is connected at the level of its lateral extremities to an upper wall of the shell 16. The appendix defines, together with the shell 16, a slit for the passage of a band 17 made of elastic material which is wound around the body of the patient 2.

The transducer 15 is protected by an outer casing. In the case of a dual transducer 15, the vibrations produced by the transmitter transducer must be prevented from being transmitted to the receiver transducer via the shell 16. For this reason, the two seats for housing the transmitter transducer and the receiver transducer respectively are physically separated from each other.

In addition, to further reduce the acoustic impedance between the transducer 15 and the esophagus 4 of the patient, a layer of gel is applied between the shell 16 and the thorax of the patient 2 to prevent the passage of air, which has a very high acoustic impedance, in this space.

Analysis of the doppler signal which strikes the transducer 15 is performed by sound identification algorithms. Analysis of the signals obtained from the quadrature demodulation of the doppler signal allows identification of both the components of the reflecting elements moving close to the transducer 15 and the components of the reflecting elements moving away from the transducer 15. In this way the analysis is partially redundant, but allows discrimination between gastro-esophageal reflux (in the direction indicated by the arrow 5) and deglutition (in the direction opposite to gastro-esophageal reflux).

The quadrature demodulation that allows identification of the direction of the reflecting elements (i.e. distinction between passage of bolus due to deglutition or due to gastro-esophageal reflux) is performed in two successive steps. In particular, the signal coming from the transducer 15 is acquired and sent to a mixer which receives at input also the carrier signal. The signal at the mixer output is then sent to a first-order filter of the low-pass type. Lastly the filtered signal is demodulated in quadrature. The quadrature demodulation step takes place in a second stage, i.e. only when the data processing unit 14, provided with specific data management and processing software, extracts, analyses and processes the data that have been collected over the twenty-four hour period in the portable memory unit 13. It should be noted that, by performing the demodulation step in a second stage, it is possible to reduce the number of electronic components necessary on the recording unit 8 and limit the consumption of the recording unit 8. Furthermore, sampling of the signals not completely demodulated does not entail any overload due to the volume of data to be saved and the sampling frequency.

The recording unit 8 is equipped with an internal controller which implements various consumption optimisation algorithms for switching to low consumption mode during the phases of inactivity. The low consumption modes also comprise the electronics for conditioning of the transducer 15 which can be enabled and disabled.

In order to perform examinations lasting twenty-four hours, it is also important in the design of the conditioning electronics for the transducer 15 (single or dual) and recording unit 8 to use very low consumption components to minimise consumption.

The long duration of the examination and the large amount of data to be analysed make it essential to carry out an analysis that is able to automatically identify all the events of interest, or any type of passage of material in the esophagus 4 of the patient 2.

In this regard it should be remembered that the esophagus 4 is located in the thoracic cavity, where other organs such as the heart, the lungs and vessels such as the aorta contribute to generating signals that interfere with the signal. In particular, the positioning of the transducer 15 immediately below the xiphoid process means that the ultrasound beam passes not only through the esophagus 4 but also through the aorta, superimposing the information on the flow of material in the esophagus on the information concerning the blood flow in the aorta.

In an initial phase, the signal is filtered to reduce the superfluous noise and components.

The initial filtering phase is performed by means of a low-pass low-order filter which makes the signal less susceptible (the filter is preferably a filter of type H(z) = 1 - a*z⁻¹, with 0.9 ≤ a < 1.0).

In a preliminary setting phase, a threshold value is also determined (said threshold value can be identified as the size of the signal in time or as the energy of the power spectrum). The threshold value represents a value extremely suitable for separating everything that is not considered silence from what is considered silence. In particular, everything below the threshold value is considered noise.

A fast Fourier transform - FFT is applied to the signals below the threshold value using the Hamming window function. For each window an LPC (Linear Predictive Coding) analysis is subsequently carried out, of order p, with 8 ≤ p ≤ 18.

The result of the LPC analysis is a series of vectors of order p characteristic of each window. The vectorial representation that can be obtained by means of LPC analysis does not entail an excessive computational burden and provides a compressed representation of the sound signal.

In a second stage the signal is identified and compared with known patterns, which are acquired by the algorithm in a preliminary setting phase. According to the result of the comparisons made with the known patterns, the decisional algorithm then assigns to each sound acquired the corresponding event (i.e. deglutition, reflux and heartbeat).

The comparison between the sound window vectors to be analysed and the known patterns can be made, alternatively, by means of a deterministic method (or template model) or by means of a statistical method.

Both methods comprise an initial phase of set-up and learning of the characteristic sound patterns to be identified by statistical or deterministic representation.

In the case of the deterministic model, at the end of the learning phase, identification maps are created, each of which contains the patterns of one specific sound to be recognised. The signal to be recognised is compared, each time, with all the identification maps, and for each comparison a distortion value is calculated. The distortion value is in turn compared with a tolerance value (which has been determined in a preliminary setting phase) and if the distortion value is below the tolerance value, the type of sound is identified.

In the statistical model, Markov chains are created for each type of sound to be identified. The probability associated with each state of the Markov chain is calculated alternatively in a discrete manner (Vector Quantization - VQ) or in a continuous manner (Guassian Mixture Model - GMM).

The characteristic vectors of the signal to be recognised, in this case, represent a sequence of states within the chain. For each Markov chain, the probability that the sequence of the signal to be recognised represents the sequence of the chain is calculated. The value with highest probability identifies the type of sound.

According to a preferred variation, operation can be of the open loop type. According to a further variation, closed loop operation is also possible for self-learning of the algorithm.

According to a first variation not illustrated, the recording unit 8 is connected to a number of cardiac derivations (i.e. to electrodes according to the known cardiac Holter technique) and to a deglutition transducer. In this way it is possible to simultaneously monitor and record, in a non-invasive manner, cardiac activity, gastro-esophageal reflux and deglutition activity.

In a further variation not illustrated, the recording unit 8 is also connected to a pulse oximeter sensor which monitors the oxygen saturation of the blood and the cardiac frequency. This embodiment can be applied in particular in the paediatric and neonatal field where it is suspected that gastro-esophageal reflux can cause apnea, i.e. cessation of respiratory activity in infants.

According to a further variation not illustrated, the monitoring unit 7 consists of a plurality of ultrasound probes 9 arranged in contact with the thorax of the patient 2 which can be configured, alternatively, in series or parallel.

The portable device 1 of ambulatory type for the diagnosis of gastro-esophageal reflux disease described so far has numerous advantages.

Firstly it is not invasive for the patient 2 who has to undergo the diagnostic examination for twenty-four hours. The use of the portable device 1 for the diagnosis of gastro-esophageal reflux disease is therefore particularly advantageous for general diagnostic screening and above all in the paediatric or neonatal field and, in general, for all those patients 2 who have difficulty tolerating the application and removal of nasogastric probes which are very invasive and uncomfortable, but widely used in the known diagnostic methods for gastro-esophageal reflux pathologies.

## Claims

1. A portable device (1) for the diagnosis of the gastro-esophageal reflux disease of a patient (2) comprising:
a monitoring unit (7) comprising at least one ultrasound probe (9) which is adapted to be externally applied to the patient (2) in the vicinity of the lower esophageal sphincter (10) of the patient (2) for continuously monitoring the flow of material through the lower esophageal sphincter (10) of the patient (2) for a predetermined number of hours no lower than twenty-four;
a portable data storage unit (8), which is connected to the monitoring unit (7) so as to collect the data detected by the monitoring unit (7); and
a data processing unit (14) for processing the signals transmitted by the probe (9) and for permitting automatic identification of the gastro-esophageal reflux, said data processing unit (14) comprising a filter and a device for receiving the filtered signal, comparing it with a number of known patterns, each of which is associated with a predetermined noise, and for associating a predetermined noise with the filtered signal.

2. Device as claimed in claim 1, wherein the probe (9) comprises a transducer (15) which is arranged on a plane defining, with the front plane of the patient (2), an angle whose size ranges from 0° to 30°, when the probe is applied to the patient .

3. Device as claimed in claim 2, wherein the ultrasound probe (9) comprises, alternatively, a single transducer (15) or a dual transducer (15), or a receiving transducer and a transmitting transducer.

4. Device as claimed in claim 1, 2 or 3, wherein the probe (9) comprises a transducer (15) which is arranged so that, when the probe is applied to the patient, the direction of the ultrasound propagation and the forward direction of the gastro-esophageal reflux form an angle of attack (θ), whose size ranges from 30° to 60°.

5. Device as claimed in one of the preceding claims, wherein the ultrasound probe (9) is arranged to be applied in direct contact with the thorax of the patient (2) with a layer of ultrasound gel being interposed between the ultrasound probe (9) and the thorax of the patient (2).

6. Device as claimed in one of the preceding claims, wherein the portable data storage unit (8) comprises: a battery; a removable memory unit (13), located inside the storage unit (8) and on which the data collected are stored and saved; and a processor for collecting and storing the data acquired and transmitting them to the memory unit (13).

7. Device as claimed in claim 6, wherein the portable data storage unit (8) comprises a display (11) by means of which, during a preliminary setting and preparation phase, the data of the patient (2) are entered and the reference parameters of the diagnostic examination undergone by the patient (2) are set; and a user interface keypad (12), by means of which the patient (2) can signal the occurrence of particular events during the diagnostic examination that he/she is undergoing.

8. Device as claimed in one of the preceding claims and comprising a position sensor.

9. Device as claimed in one of the preceding claims, wherein the filter is of the low-pass type with low order, preferably of the first order.

## Patentansprüche

1. Tragbares Gerät (1) zur Diagnose von gastroösophagealem Reflux bei einem Patienten (2), umfassend:
eine Überwachungseinheit (7), umfassend zumindest eine Ultraschallsonde (9), die zur externen Anlage an den Patienten (2) in der Nähe des unteren Ösophagussphinkters (10) des Patienten (2) ausgestaltet ist, um den Materialfluss durch den unteren Ösophagussphinkter (10) des Patienten (2) für eine vorgegebene, nicht unter vierundzwanzig liegende Stundenanzahl kontinuierlich zu überwachen;
eine tragbare Datenspeichereinheit (8), die mit der Überwachungseinheit (7) so verbunden ist, um die von der Überwachungseinheit (7) detektierten Daten zu sammeln; und
eine Datenverarbeitungseinheit (14), um die von der Sonde (9) übertragenen Signale bearbeiten und um eine automatische Identifizierung des gastroösophagealen Refluxes zu ermöglichen, wobei die Datenverarbeitungseinheit (14) einen Filter und ein Gerät zum Empfangen des gefilterten Signals umfasst, es mit einer Anzahl bekannter Muster vergleicht, die jeweils zu einem vorgegebenen Störgeräusch gehören, und ein vorgegebenes Störgeräusch mit dem gefilterten Signal assoziiert.

2. Gerät nach Anspruch 1, wobei die Sonde (9) einen Wandler (15) umfasst, der auf einer Ebene angeordnet ist, welche mit der vorderen Ebene des Patienten (2) einen Winkel definiert, dessen Größe von 0 ° bis 30 ° reicht, wenn die Sonde an den Patienten angelegt ist.

3. Gerät nach Anspruch 2, wobei die Ultraschallsonde (9) wahlweise einen Einzelwandler (15) oder einen Doppelwandler (15) oder einen Empfangswandler und einen Übertragungswandler umfasst.

4. Gerät nach Anspruch 1, 2 oder 3, wobei die Sonde (9) einen Wandler (15) umfasst, der so angeordnet ist, dass bei Anliegen der Sonde am Patienten die Richtung der Ultraschallausbreitung und die Vorwärtsrichtung des gastroösophagealen Refluxes einen Angriffswinkel (θ) ausbilden, dessen Größe zwischen 30° und 60° liegt.

5. Gerät nach einem der vorherigen Ansprüche, wobei die Ultraschallsonde (9) so angeordnet ist, dass sie in direktem Kontakt mit dem Thorax des Patienten (2) angelegt ist, wobei sich zwischen der Ultraschallsonde (9) und dem Thorax des Patienten (2) eine Schicht Ultraschallgel befindet.

6. Gerät nach einem der vorherigen Ansprüche, wobei das tragbare Datenspeichereinheit (8) umfasst: eine Batterie, eine entfernbare Speichereinheit (13), die innerhalb der Datenspeichereinheit (8) liegt und auf der die gesammelten Daten sicher gespeichert sind; und einen Prozessor zum Sammeln und Speichern der erworbenen Daten und zu deren Übertragung an die Bildspeichereinheit (13).

7. Gerät nach Anspruch 6, wobei die tragbare Datenspeichereinheit (8) umfasst:
eine Anzeigevorrichtung (11), mit deren Hilfe die Daten des Patienten (2) während einer Voreinstellungs- und Vorbereitungsphase eingegeben werden und die Bezugsparameter der bei dem Patienten (2) vorgenommenen diagnostischen Untersuchung eingestellt werden; und
eine Benutzer-Schnittstellen-Tastatur (12), mit deren Hilfe der Patient (2) das Auftreten besonderer Vorkommnisse während der an ihm/ihr vorgenommenen diagnostischen Untersuchung signalisieren kann.

8. Gerät nach einem der vorherigen Ansprüche, das einen Positionssensor umfasst.

9. Gerät nach einem der vorherigen Ansprüche, wobei es sich bei der Filterart um einen Tiefpassfilter geringer Ordnung, bevorzugt erster Ordnung, handelt.

## Revendications

1. Dispositif portable (1) pour le diagnostic du reflux gastro-oesophagien d'un patient (2) comprenant :
une unité de surveillance (7) comprenant au moins une sonde ultrasonore (9) qui est conçue pour être appliquée extérieurement au patient (2) dans le voisinage du sphincter oesophagien inférieur (10) du patient (2) pour surveiller continûment le flux de matière à travers le sphincter oesophagien inférieur (10) du patient (2) pendant un nombre prédéterminé d'heures qui n'est pas inférieur à vingt-quatre ;
une unité de mémorisation de données (8) portable, qui est connectée à l'unité de surveillance (7) de manière à collecter les données détectées par l'unité de surveillance (7) ; et
une unité de traitement de données (14) pour traiter les signaux transmis par la sonde (9) et pour permettre une identification automatique du reflux gastro-oesophagien, ladite unité de traitement de données (14) comprenant un filtre et un dispositif pour recevoir le signal filtré, le comparer avec un certain nombre de modèles connus, qui sont associés chacun à un bruit prédéterminé, et pour associer un bruit prédéterminé au signal filtré.

2. Dispositif selon la revendication 1, dans lequel la sonde (9) comprend un transducteur (15) qui est agencé dans un plan définissant, avec le plan avant du patient (2), un angle dont la taille va de 0° à 30°, lorsque la sonde est appliquée au patient.

3. Dispositif selon la revendication 2, dans lequel la sonde ultrasonore (9) comprend, en variante, un transducteur (15) unique ou un transducteur (15) double, ou un transducteur de réception et un transducteur d'émission.

4. Dispositif selon la revendication 1, 2 ou 3, dans lequel la sonde (9) comprend un transducteur (15) qui est agencé de sorte que, lorsque la sonde est appliquée au patient, la direction de la propagation d'ultrasons et la direction avant du reflux gastro-oesophagien forment un angle d'attaque (θ), dont la taille va de 30° à 60°.

5. Dispositif selon l'une des revendications précédentes, dans lequel la sonde ultrasonore (9) est agencée pour être appliquée en contact direct avec le thorax du patient (2), une couche de gel pour ultrasons étant interposée entre la sonde ultrasonore (9) et le thorax du patient (2).

6. Dispositif selon l'une des revendications précédentes, dans lequel l'unité de mémorisation de données (8) portable comprend : une batterie ; une unité de mémorisation (13) amovible, située à l'intérieur de l'unité de mémorisation (8) et dans laquelle les données collectées sont mémorisées et sauvegardées ; et un processeur pour collecter et mémoriser les données acquises et les transmettre à l'unité de mémorisation (13).

7. Dispositif selon la revendication 6, dans lequel l'unité de mémorisation de données (8) portable comprend un afficheur (11) au moyen duquel, pendant une phase préliminaire de réglage et de préparation, les données du patient (2) sont entrées et les paramètres de référence de l'examen de diagnostic subi par le patient (2) sont réglés ; et un pavé numérique d'interface utilisateur (12), au moyen duquel le patient (2) peut signaler l'apparition d'événements particuliers pendant l'examen de diagnostic qu'il subit.

8. Dispositif selon l'une des revendications précédentes et comprenant un capteur de position.

9. Dispositif selon l'une des revendications précédentes, dans lequel le filtre est du type passe-bas avec un faible ordre, de préférence du premier ordre.
